# EUROPEAN PATENT SPECIFICATION

(11) **EP 4 531 963 B1**
(45) Date of publication and mention of the grant of the patent: **11.11.2026**
(21) Application number: 22843635.8
(22) Date of filing: 20.12.2022
(51) Int. Cl.: A61M 5/315

(54) **PISTON EXTENSION ROD FOR INJECTOR PISTON MOUNTING**
KOLBENVERLÄNGERUNGSSTANGE ZUR INJEKTORKOLBENLAGERUNG
TIGE D'EXTENSION DE PISTON POUR MONTAGE DE PISTON D'INJECTEUR

(30) Priority: 28.05.2022 DK PA202200497; 01.06.2022 DK PA202200516; 27.07.2022 DK PA202200720
(43) Date of publication of application: 09.04.2025
(73) Proprietor: Injecto Group A/S, 2900 Hellerup (DK)
(72) Inventor: HETTING, Mikael, 2920 Charlottenlund (DK)
(74) Representative: AWA Denmark A/S
(86) International application number: PCT/DK2022/050297
(87) International publication number: WO 2023/232208

(56) References cited:
- US-A- 3 834 387
- US-A1- 2021 046 248
- US-A1- 2021 128 840

## Description

### Field of the invention

The present invention relates to a piston mounting method for an injector, which mounting method enables the positioning of the piston inside an injector's gas-filled compartment by its mechanical interaction with the piston. The invention and is intended for optimized mounting of pistons in prefilled syringes (PFS) and cartridges for delivery of pharmaceutical compositions, such as a vaccine or biologic.

### Background

Known injectors are in many cases constructed so that they can be filled, and a piston mounted by automatic fill and mounting equipment. Piston mounting relates to methods concerning mounting of the piston, which are done by different methods depending on the type of syringe in question. By one example traditional disposable syringes typically comprise a container, a piston and a piston rod, which piston is mounted on the piston rod before the piston rod with piston is inserted into the container in order to reach the inside bottom portion of the container by the container outlet, or the piston may be mounted in the container followed by mounting of the piston rod..

By another example pistons for prefilled syringes (PFS) are mounted after filling of the container which is done by either vacuum or mechanical mounting, both designed to allow the piston to enter the PFS and be positioned close to the drug without physical resistance from the air present between the drug upper surface and the container opening, which will compress and prevent the piston to be mounted appropriately, which is the challenge to overcome during piston mounting in drug-filled PFS. Mechanical piston mounting, also known as vent-tubing, was the first method introduced for piston mounting of PFS, and is done by significant compression of the piston followed by insertion in a tube, through which tube the piston is forced into the correct position in the PFS. This method ensures that the excess air inside the PFS can bypass through the gap between the container inner wall and the tube outside wall, and the piston can be positioned with a residual amount of air typically in the magnitude of 1 to 5 mm.

Vacuum piston mounting was developed as an alternative to the taxing vent-tubing, where the severe compression and force involved can often result in damage to the piston sealing elements and damage the poly-tetrafluoro ethylene (PTFE) coating used for more advanced pistons for sensitive biologic formulations. Furthermore, vent-tubing has compatibility issues with silicone-free applications, since the mounting and acceleration of the piston cannot be performed through the vent-tube without considerable damage to the piston sealing elements due to excess friction and significant temperature increase without the addition of lubrication, normally silicone.

Vacuum piston mounting is done by automated equipment including a vacuum pump creating a vacuum in the container between the PFS finger flange and the drug upper surface, which space defines the volume of air, which when converted to sufficient vacuum, draws the piston into the container and further into the intended position just above the drug upper surface. In contradiction to vent-tubing, vacuum mounting enables a mounting with less residual air and even so-called *bubble-free* filling, which is advantageous for certain oxygen sensitive drug applications. For larger containers and special applications with smaller volumes relative to the container size, vacuum alone may not be sufficient to draw the piston to the intended position, and therefore assisted vacuum mounting is utilized in such cases. Assisted vacuum mounting combines vacuum mounting with physical support of a pushing rod for placement of the piston.

Assisted vacuum is also used for silicone free PFS applications where there is no lubrication of the piston and container resulting in a higher friction coefficient than for a siliconized PFS.

The existing mounting methods are well known and utilized within the pharma industry. However, there is a number of scenarios and applications where the described stoppering technologies do not suffice.

A challenge with vacuum mounting emerges when the fill volume is substantially below the total physical volume of the container in use. By example an ophthalmic drug injectable volume of between 0.05 and 0.165 ml is done from a 0.5 ml container body and leaves a residual volume of air of between approximately 0.49 ml and 0.34 ml, which configuration requires a significant amount of vacuum to move the piston the distance from container opening and down to the surface of the ophthalmic drug. With increased vacuum there is a real risk of the drug being exposed to excess temperature and even boiling, rendering the drug less efficient or even damaged. At the same time ophthalmic drugs must never be exposed to silicone, why the combination of a small fill volume and silicone free application calls for a mounting method which accommodates both challenges.

US 5,411,489 discloses pre-filled syringes and pre-filled cartridges for administering various fluids into a patient. The objective of US 5,411,489 is to address problems relating to inadequate sliding properties in pre-filled syringes stored for extended time periods caused by the need to obtain a good leakproof seal. The syringe comprises a barrel; a cup-shaped plunger, a plunger actuating cylinder and a plunger rod. The plunger rod of US 5,411,489 has a tip at a distal end with a convex face, a knob at the proximal end located outside the plunger actuating cylinder, and a flange also at the proximal end. The plunger rod is configured to be inserted into the plunger actuating cylinder and push the inside wall of the cup-shaped plunger and deform the plunger. The syringe is filled with a medicament or the like from the tapered tip, where a hypodermic needle can be mounted, after inserting the plunger into the syringe barrel.

WO 2019/199901 discloses plungers and their use in drug delivery devices, such as pre-filled syringes, cartridges or auto-injectors. The drug delivery device has a plunger that may be in an "expanded state" or "storage mode" and which may be changed to be in a "constricted state" or "dispensing mode". The plunger rod for the drug delivery device has an axial protrusion, which is inserted into a cavity in the plunger, and when sufficient distal force is applied via the plunger rod, this causes the axial protrusion to apply force in a distal direction onto an engagement surface in the cavity. The plunger then axially elongates along a stretch zone causing the plunger to slightly constrict about the stretch zone, and constriction of the plunger reduces radial compression onto the sidewall of the medical barrel, thus allowing that the plunger may be more easily advanced down the medical barrel while maintaining a liquid tight seal and container closure integrity.

WO 2019/185101 discloses an injector having a stopper with a cavity, the location and design of which provides a reduced break loose force (BLF) for the injector compared to an injector having a stopper without the cavity.

US3834387 discloses a syringe with a plunger assembly permitting the filling of the syringe through its breech and a method of introducing fluid into the breech end of a syringe and for installing the plunger and piston assembly therein. The syringe is filled with a liquid medicament, and a control rod stretches or extends the piston to deform it allowing the plunger to be easily moved within the chamber without trapping and compressing air or creating a suction force.

Within the field of pre-filled syringes there is a need for a simplified procedure to mount a piston in a syringe, which has a decreased risk of damaging the liquid drug in the syringe. The present invention aims to address this need.

### Summary

The invention is met by providing a novel mounting method for a piston in a prefilled syringe (PFS). The method comprises the steps of:
providing an injector comprising a cylinder having a longitudinal axis and an inner wall, and a liquid drug defining a drug upper surface,
providing a piston having a deformable sealing element and a cavity extending from a piston cavity opening to a piston cavity base to define a total piston cavity length,
inserting the piston in the cylinder so that the deformable sealing element abuts the inner wall of the cylinder and seals an annular gap between the piston and the inner wall of the cylinder,
providing a piston extension rod having a total length of at least 5% more than the total cavity length, and an impact surface,
inserting the piston extension rod in the cavity of the piston and impacting the piston cavity base with the impact surface at a velocity of at least 400 mm/min to extend the piston along the cylinder longitudinal axis and cause a contraction of the deformable sealing element to create a bypass for air between the deformable sealing element and the inner wall of the cylinder, and
maintaining the velocity of the piston extension rod to move the piston to a final piston position.

For example, the invention provides a method of mounting a piston in a prefilled injector, the method comprising the steps of:
providing an injector comprising a cylinder having an inner wall and an inner diameter and an outlet at an outlet end opposite an actuating end, the cylinder comprising a liquid drug defining an upper surface; and
providing a piston having a piston body with an actuating surface opposite an outlet surface, an axial length between the actuating surface and the outlet surface, and a transverse diameter, which piston body defines a piston cavity opening having an access diameter, the piston at an axial location from the actuating surface comprising a deformable sealing element surrounding the stopper body and having an outer diameter, which is larger than the transverse diameter, and
which piston comprises a piston cavity extending from the piston cavity opening to a base of the piston cavity, the piston cavity defining a total cavity length,
providing a piston extension rod having a diameter equal to or smaller than the access diameter and a length at least 5% larger than the total cavity length, the piston extension rod having an impact surface at an end of the piston extension rod,
inserting the piston in the cylinder at the actuating end so that the deformable sealing element seals an annular gap between the stopper body and the inner wall of the cylinder,
inserting the piston extension rod in the piston cavity opening and impacting the impact surface with the base of the piston cavity at a velocity of at least 400 mm/min,
moving the piston extension rod in a longitudinal direction from the actuating end of the cylinder to the outlet end of the cylinder to contract the deformable sealing element and create a bypass for air between the deformable sealing element and the inner wall of the cylinder, and
maintaining the velocity of the piston extension rod to move the piston to a final piston position. For example, moving the piston extension rod in the longitudinal direction from the actuating end of the cylinder to the outlet end of the cylinder causes extension of the piston in the longitudinal direction to contract the deformable sealing element and create a bypass for air between the deformable sealing element and the inner wall of the cylinder.

In the methods, a piston is mounted in a prefilled syringe. In the present context, "mounting" may also be referred to as "inserting", and the two terms may be used interchangeably. Furthermore, the "piston" may also be referred to as a "stopper", and the two terms may be used interchangeably. The piston cavity base is impacted with the impact surface of the piston extension rod at a velocity of at least 400 mm/min. It is to be understood that the piston extension rod is commonly accelerated from no velocity to the velocity of impact and therefore the velocity may also be referred to as an "accelerated movement", and the two terms may be used interchangeably in the present context. The piston extension rod may also be abbreviated PER, and the two terms may be used interchangeably in the context of the present disclosure. The piston has a piston cavity opening. The "piston cavity opening" may also be referred to as a "cavity entrance" or a "piston cavity entrance", and the terms may be used interchangeably in the context of the present disclosure. In general, the piston cavity opening may have an access diameter. Thus, for example, the method may also be considered to be a method of inserting a piston into a cylinder, the method comprising the steps of:
providing an injector comprising a cylinder having an inner wall and an inner diameter and an outlet at an outlet end opposite an actuating end, and
a stopper having a stopper body with an actuating surface opposite an outlet surface, an axial length between the actuating surface and the outlet surface, and a transverse diameter, which stopper body defines a cavity entrance having an access diameter,
the stopper at an axial location from the actuating surface comprising a deformable sealing element surrounding the stopper body and having an outer diameter, which is larger than the transverse diameter, and
the stopper comprising a piston cavity extending from the cavity entrance to a base of the piston cavity, the piston cavity defining a total cavity length,
providing a piston extension rod having a diameter equal to or smaller than the access diameter and a length at least 5% larger than the total cavity length, the piston extension rod having an impact surface at an end of the piston extension rod,
inserting the piston in the cylinder at the actuating end so that the deformable sealing element seals an annular gap between the stopper body and the inner wall of the cylinder,
inserting the piston extension rod in the cavity entrance and impacting the impact surface with the base of the piston cavity at an accelerated movement of at least 400 mm/min,
moving the piston extension rod in a longitudinal direction from the actuating end of the cylinder to the outlet end of the cylinder to deform the deformable sealing element and create a bypass for air between the deformable sealing element and the inner wall of the cylinder. For example, the piston may be moved to a final piston position.

The step of inserting the piston in the cylinder may employ a dedicated tool. For example, prior to being inserted in the cylinder, the piston may be preinserted in an insertion tube. The insertion tube may have an inner diameter, which is identical to the inner diameter of the cylinder, or which is slightly smaller or larger than the inner diameter of the cylinder. For example, the inner diameter of insertion tube may be in the range of 90% to 110% of the inner diameter of the cylinder. The length of the insertion tube is generally in the range of 80% to 200%, e.g. 100% to 150%, of the length of the piston. By using an insertion tube, insertion of the piston in the cylinder can be performed faster than when no insertion tube is used, and thereby a faster process for mounting pistons in prefilled syringes is provided.

When the piston has been inserted in the cylinder, the piston extension rod is inserted in the cavity of the piston, and the piston cavity base is impacted with the impact surface of the piston extension rod at a velocity of at least 400 mm/min. This velocity ensures that the piston is extended along the cylinder longitudinal axis and at the same time it causes a contraction of the deformable sealing element, which in turn creates a bypass of air in the space between the piston and the drug upper surface. Thereby, the piston can be moved closer to the drug upper surface, and air can be removed between the drug upper surface and the piston during the movement of the piston to the final piston position. Besides the ability to bypass air from a PFS the invention introduces further derived significant advantages. The contraction of the sealing elements enables a low friction without the usual strain and stress on sealing elements known from existing piston mounting systems. The method renders vacuum redundant, including costs related to invest in and operate the vacuum system.

The mounting speed can be significantly increased due to low friction mounting, hence enabling a larger annual output per time unit. The velocity may for example be in the range of 400 mm/min to 120,000 mm/min. Once the piston cavity base has been impacted with the impact surface, the velocity need not be constant during the movement of the piston to the final piston position, and the velocity may be varied in the range of 400 mm/min to 120,000 mm/min, e.g. the velocity may be in the range of 400 mm/min to 20,000 mm/min, e.g. 400 mm/min to 10,000 mm/min, 400 mm/min to 5,000 mm/min, 400 mm/min to 2,000 mm/min or 400 mm/min to 800 mm/min. The lower power consumption and higher output per time unit has a significant positive impact on the CO₂ operating profile compared to both vent-tubing but also vacuum mounting. The appropriate piston extension rod speed may be chosen for each piston characteristic and PFS configuration and the piston extension rod speed range is at least 400mm/min and up to maximum speeds of the automated filling and mounting systems on which the piston extension rod is mounted.

The cylinder has an inner wall, and the cylinder may further be defined with an inner diameter. However, in the present context, the term "diameter" does not imply that the corresponding element must have a circular cross-section, and any cross-sectional shape as desired may be used for the corresponding element. Thus, the cross-section of the cylinder may be polygonal, e.g. triangular, square, pentagonal, hexagonal, etc., and the term diameter will in this case refer to a cross-sectional dimension, e.g. the largest cross-sectional dimension for the corresponding cross-sectional shape. Correspondingly, the cavity opening and the piston extension rod are not limited to be round but may have any shape as desired, e.g. piston extension rod and also the cavity opening may have a cross-section that is polygonal, e.g. triangular, square, pentagonal, hexagonal, etc., and the term diameter will in this case refer to a cross-sectional dimension, e.g. the largest cross-sectional dimension for the corresponding cross-sectional shape. A polygonal cross-section is not limited to polygons having equal angles and side lengths, i.e. regular polygons, and likewise the cross-section may also be elliptical. The piston extension rod dimension may also be asymmetric in its longitudinal axis suitable for interaction with a piston cavity.

The piston may be defined to have a piston body. In general, the piston body does not interact with the inner wall of the cylinder, and the cross-sectional shape of the piston body can be chosen freely, regardless of the cross-sectional shape of the deformable sealing element. The piston body generally has a transverse diameter, which is smaller than the inner diameter of the cylinder. The piston has a deformable sealing element. The deformable sealing element is configured to abut the inner wall of the cylinder and seal an annular gap between the piston and the inner wall of the cylinder. Thus, the deformable sealing element generally surrounds the stopper body and has an outer diameter, which is larger than the inner diameter of the cylinder. For example, the outer diameter of the deformable sealing element may be 1.5% to 10% larger, e.g. 2% to 5% larger, than the inner diameter of the cylinder. When the outer diameter of the deformable sealing element is at least 1.5% larger than the inner diameter of the cylinder, especially when the deformable sealing element has a Shore A hardness in the range of 40 to 75, the container closure integrity (CCI) is ensured.

The piston extension rod may be mounted on a connector. In particular, the piston extension rod may extend from a connector. The connector has a size allowing it to be inserted into the cylinder during mounting of the piston in the injector, e.g. the connector has a smaller diameter than the inner diameter of the cylinder. The connector may have any length as appropriate. For example, the combined length of the connector and the piston extension rod may be sufficient to move the piston to a final piston position where the outlet surface of the piston is in contact with the liquid drug in the cylinder, e.g. at the drug upper surface of the liquid drug.

The piston has a piston cavity opening. The piston may be described to have an actuating surface opposite an outlet surface, where the piston cavity opening is located in the actuating surface. The actuating surface and the outlet surface are located at opposite ends, i.e. opposite ends in an axial dimension, of the piston body. When inserted in the cylinder, the axial dimension of the piston body substantially coincides with the longitudinal axis of the cylinder. The actuating surface may also be said to be at an actuating end of the piston body, and the outlet surface may also be said to be at an outlet end of the piston body. When inserted into the cylinder of an injector, the outlet end of the piston body faces the outlet of the injector.

The outlet surface of the piston is the surface facing the liquid drug in the cylinder of the injector. In an example, the outlet surface of the piston is in contact with the liquid drug in the cylinder at the final piston position. Thereby, the method allows that there is substantially no residual air in the prefilled injector between the piston and the liquid drug. This is particularly advantageous for ophthalmic drugs where the volume for injection is typically very small, e.g. between 0.05 ml and 0.165 ml in a cylinder with a nominal dosis of 0.5 ml.

The piston has a cavity. The cavity may also be referred to as a "piston cavity", and the two terms may be used interchangeably. The cavity is configured to receive the piston extension rod but is otherwise not limited with respect to its shape. The cavity and a piston rod for use with the injector may include an engagement device and a complementary engagement device, respectively, and the engagement device and the complementary engagement device may be chosen freely. For example, a piston rod for use with the injector may include an external thread, e.g. a helical external thread, and the cavity may correspondingly comprise a complementary internal thread, e.g. a helical internal thread, the threads thus providing the engagement device and the complementary engagement device, respectively.

In an example, the cavity has a generally cylindrical shape. For example, the cavity may have a diameter substantially equal to the access diameter. In another example, the cavity has a generally conical shape with the diameter of the cavity narrowing from the cavity opening toward the piston cavity base.

In a specific example, the piston has a cavity as defined in WO 2019/185101. Thus, for example, the piston may comprise a deformable sealing element at an axial location from the piston cavity opening, e.g. from the actuating surface, and a broadened cavity section at the axial location of the deformable sealing element, which broadened cavity section has an axial extension in the range of 5% and 70% of the total piston cavity length, e.g. in the range of 5% and 50% of the total piston cavity length and which broadened cavity section, e.g. over its axial extension, has a lateral extension that is larger than the piston cavity opening, e.g. the access diameter. This example with the broadened cavity section may also be referred to as a BLF-reducing cavity. In an example, the lateral extension of the broadened cavity section, e.g. over the axial extension of the broadened cavity section, is at least 50% of the outer diameter of the deformable sealing element and larger than the piston cavity opening, e.g. the access diameter. In examples of the disclosure, the broadened cavity section has a lateral extension that is up to 90%, e.g. up to 80%, up to 70% or up to 60% of the of the outer diameter of the deformable sealing element. For example, the piston may have a piston body with an actuating surface opposite an outlet surface, an axial length between the actuating surface and the outlet surface, and a transverse diameter, and which piston, at an axial location from the actuating surface, comprises a deformable sealing element, e.g. made from a thermoplastic elastomer (TPE), which deformable sealing element surrounds the piston body and has an outer diameter, which is larger than the transverse diameter, e.g. which deformable sealing element has an axial extension in the range of 5% and 95% of the axial length of the piston body, and which deformable sealing element seals an annular gap between the piston body and the inner wall of the cylinder, when the piston is inserted into the cylinder, and which piston comprises a broadened cavity section at the axial location of the deformable sealing element, the broadened cavity section having an axial extension in the range of 5% and 50% of the total piston cavity length, and which broadened cavity section, e.g. over its axial extension, has a lateral extension that is larger than the piston cavity opening, e.g. the access diameter, and in the range of 50% to 90% of the outer diameter of the deformable sealing element.

The piston, e.g. the piston body, may have a tubular section for housing a piston rod or for receiving the piston extension rod, which tubular section extends from the piston cavity opening, e.g. from the actuating surface of the piston, to the broadened cavity section. When the piston comprises a tubular section, the piston cavity opening may have an access diameter. Specifically, when an injector with this example of the piston is fitted with a piston rod, a cavity may be formed at an interface between the piston body and the piston rod and/or at an interface between the deformable sealing element and the piston rod at the time the injector is ready for use. In general, the broadened cavity section has a lateral extension that is at least 50% of the outer diameter of the deformable sealing element and larger than the access diameter. In particular, the broadened cavity section is larger than the access diameter. When the piston comprises a broadened cavity section at the axial location of the deformable sealing element as defined from the actuating surface, i.e. when there is an overlap in the axial location of the deformable sealing element and the cavity, and when the cavity has a diameter, which is larger than the access diameter, especially when the diameter of the cavity is in the range of 50% to 90%, e.g. 60% to 80%, of the outer diameter of the deformable sealing element, the formation of the bypass of air is obtained more easily than when the piston comprises a cavity of a smaller size. This is particularly relevant, when the velocity is relatively low, e.g. in the range of 400 mm/min to 800 mm/min. The effect is also especially relevant when the deformable sealing element has a Shore A hardness in the range of 40 to 75, and even more relevant when the deformable sealing element is made from a TPE, e.g. when the piston has been injection moulded from a TPE. Furthermore, when the injector comprises a broadened cavity section as defined above, i.e. when the cavity has a diameter, which is larger than the access diameter, especially when the diameter of the cavity is in the range of 50% to 90%, e.g. 60% to 80%, of the outer diameter of the deformable sealing element, and when the cavity has an axial extension in the range of 5% and 50% of the total piston cavity length, and when the tubular section for housing a piston rod or for receiving the piston extension rod has the access diameter, an injector fitted with the piston and an appropriate piston rod will have a decreased break loose force (BLF) compared to an injector having a piston where the cavity does not comprise the broadened or BLF-reducing cavity section.

In another aspect, the invention relates to a kit of parts comprising an injector comprising a cylinder having a longitudinal axis and an inner wall, e.g. a cylinder having an inner diameter; a piston having a piston body with an actuating surface opposite an outlet surface, and a transverse diameter, which piston, at an axial location from the actuating surface, comprises a deformable sealing element surrounding the piston body and having an outer diameter, which is larger than the transverse diameter, the piston having a cavity extending from a piston cavity opening, i.e. a piston cavity opening in the actuating surface, to a piston cavity base to define a total piston cavity length, the cavity comprising a tubular section extending from the piston cavity opening, i.e. from the actuating surface, and having an access diameter and a broadened cavity section at the axial location of the deformable sealing element and having an axial extension in the range of 5% and 50% of the total piston cavity length, and which broadened cavity section, e.g. over its axial extension, has a lateral extension that is larger than the access diameter and in the range of 50% to 90% of the outer diameter of the deformable sealing element, e.g. up to 80%, up to 70% or up to 60% of the of the outer diameter of the deformable sealing element, and a piston extension rod having a diameter equal to or smaller than the access diameter and a length at least 5% larger than the total cavity length, e.g. the piston extension rod extends from a connector. The piston may for example have an axial length between the actuating surface and the outlet surface. The deformable sealing element is generally configured to seal an annular gap between the piston body and the inner wall of the cylinder, when the piston is inserted into the cylinder.

The kit of parts may comprise any additional elements, such as actuators, processor units and the like, to perform the method of the invention. In an example, the deformable sealing element has a Shore A hardness in the range of 40 to 75. For example, the deformable sealing element may be made from a TPE, e.g. the piston may be injection moulded from a TPE, e.g. as a single piece. In an example, the piston extension rod comprises a backstop located at an end opposite the impact surface. The backstop has a lateral size relative to the piston extension rod, which is larger than the piston cavity opening, e.g. the access diameter. The lateral size of the backstop is smaller than the inner diameter of the cylinder. Thereby, it is prevented that the piston is pushed too far into the cylinder of the prefilled injector. For example, the piston extension rod may have a length in the range of 10% to 30% of the total cavity length.

The tubular section can be said to be configured to receive the piston extension rod, and it generally extends from the piston cavity opening to the broadened cavity section.

In an example, the piston extension rod has a diameter, and the access diameter is in the range of 10% to 500% larger than the diameter of the piston extension rod. Furthermore, the piston extension rod may have a length that is 5% to 80% longer than the total piston cavity length, e.g. 5% to 50% longer than the total piston cavity length.

The deformable sealing element may be made from any appropriate elastomeric material. In an example, the deformable sealing element, and optionally also the piston body, is made from a TPE, e.g. by injection moulding from a TPE. Any TPE may be used for the piston of the invention, e.g. for the deformable sealing element and also the piston body. Appropriate TPEs comprise SBCs, e.g. hydrogenated - H-SBC - (SEBS - styreneethylene butylenes-styrene or similar) or non-hydrogenated (SBS - styrene-butadienestyrene) or alloys of these and other compatible polymers, such as COC elastomers, or styrene-butadiene (SB), styrene-isoprene-styrene (SIS), styrene-isoprene-butadiene-styrene (SIBS), styrene-ethylene-ethylenepropylene-styrene (SEEPS) or alloys of any of these compounds. Preferred SBCs are those known under the trademark Evoprene as marketed by AlphaGary Corporation (Leominster, MA, USA), and Mexichem Specialty Compounds. Evoprenes are described in the brochure "EVOPRENE™ Thermoplastic Elastomer (TPE) Compounds - GENERAL INFORMATION" (published by AlphaGary, July 2007), and preferred Evoprene^{™} polymers are Evoprene^{™} Super G, Evoprene^{™} G, Evoprene^{™} GC, and Evoprene^{™} HP, which are described in the brochures "EVOPRENE^{™} SUPER G Thermoplastic Elastomer (TPE) Compounds", "EVOPRENE^{™} G Thermoplastic Elastomer (TPE) Compounds", "EVOPRENE^{™} GC Thermoplastic Elastomer (TPE) Compounds", and EVOPRENE™ HP Thermoplastic Elastomer (TPE) Compounds (published by AlphaGary, July 2007), respectively.. Other relevant elastomers comprise COC elastomers, e.g. TOPAS^{®} Elastomer E-140. The TPE may be selected based on the gas, e.g. oxygen, permeability, and in general it is preferred, especially for a piston for a prefilled syringe, that the gas permeability is as low as possible. SIBS TPEs generally have very low gas permeabilities and these are therefore appropriate for pistons for prefilled syringes. Other relevant elastomeric materials include rubbers, e.g. natural rubber, synthetic rubber (polyisoprene rubber, butyl rubber, halobutyl rubber), silicone rubber, and the like, and thermoplastic vulcanisates (TPVs).

The elastomeric material can be defined with respect to its hardness, e.g. a Shore durometer, which indicates the elasticity of the elastomeric material and measures the hardness of the elastomeric material, where the higher the durometer, the harder the compound. Materials defined with a Shore A hardness are preferred, and the Shore A hardness may be in the range of 40 to 75. Measurement of the Shore A hardness is well-known to the skilled person and in particular the Shore A hardness is generally recorded according to the ISO 868 standard.

A TPE may also be defined by its compression set value, which corresponds to the deformation remaining after removal of a force that was applied to it (and is typically expressed in %). The compression set value is typically recorded over a specified period of time, e.g. in the range of 18 hours to 96 hours or 22 to 72 hours, and at a specified temperature, for example according to the ISO 815 standard. In the context of the present invention the compression set is generally recorded at an "ambient temperature", e.g. in the range of 10°C to 40°C. However, the temperature range may also extend beyond ambient temperature, e.g. 23°C to 100°C. In general, the higher the temperature the shorter the time relevant for recording the compression set. The compression set should generally be as low as possible but for a stopper, or a part of a stopper, of the invention the compression set may be in the range of 15% to 40%, e.g. at ambient temperature. At higher temperatures, e.g. 100°C, the compression set will typically be higher, e.g. up to 50%. It is, however, preferred that the compression set at ambient temperature is in the range of 10% to 40%. The compression set value is generally relevant for prefilled injectors where the stopper will be inserted into the cylinder and therefore compressed when the prefilled injector is stored for extended periods of time. When the stopper, e.g. the stopper body and also the deformable sealing element, has a Shore A hardness in the range of 30 to 90, e.g. 50 to 90, and a compression set value of at least 25%, e.g. in the range of 25% to 35%, the BLF of a prefilled injector of the invention will decrease upon storage, e.g. for at least 5 days, so that a stopper of the invention is especially advantageous for a prefilled injector.

The deformable sealing element is preferably convex. In this context, the term "convex" means that a straight line between any two points within the deformable sealing element does not cross the surface of the deformable sealing element. Any convex shape is contemplated, but the deformable sealing element preferably has a point, e.g. a point in an axial plane of the stopper, representing the maximal extension from the centre axis of the stopper.

In a specific example, the deformable sealing element is made from a TPE, e.g. with a Shore A hardness in the range of 40 to 75. This allows that the piston can be inserted in the cylinder and moved to the final piston position in the absence of an external lubricant.

The present piston mounting method enables precision mounting without vacuum, or the use of piston compression by vent-tubing. According to the preferred example, the piston extension rod can be circular cylindric along its longitudinal axis parallel to the container longitudinal axis. In other examples it can be oval, square or rectangular as needed for cooperation with a specific piston cavity. In further examples the piston extension rod may have outward protrusions or inward grooves to accommodate the interaction with a piston cavity said protrusions and or grooves established along the piston extension rod longitudinal axis. According to the invention the piston extension rod has a length along its longitudinal axis parallel to the longitudinal axis of the container exceeding the piston cavity depth along the container longitudinal axis by at least 5%. In particular, it is preferred that only the impact surface of the piston extension rod impacts the piston, i.e. at the piston cavity base. For example, the piston extension rod may not have a part or section, e.g. a backstop, that can impact the surface of the piston having the entrance of the cavity. However, the piston extension rod may also have a backstop, which can control the maximal extension of the piston during mounting. During piston mounting the piston extension rod will accelerate and enter the piston cavity. During the piston extension rod acceleration and downward movement when reaching the base of the piston cavity the acceleration of the piston extension rod will extend the piston at impact with the piston cavity base and continue the final positioning of the piston close to the drug upper surface. The piston extension is caused by the piston extension rod acceleration and impact with the stationary piston and its cavity base in combination with the elastomeric properties of the piston. At the same time as the extension said piston extension will result in a contraction of the piston perpendicular to the container longitudinal axis thereby fully or partly releasing the piston sealing element from contact with the container inner wall, ultimately creating the necessary bypass for outlet of excess air existing between the piston and drug upper surface. The contraction of the piston is a function of the piston extension and a natural consequence of the piston as an elastomeric component which will contract when extended. While the contraction is triggered by the impact from the piston extension rod and into the piston cavity base at its stationary position the contraction is maintained by a combination of piston extension rod speed during final piston positioning and the reciprocal force friction caused by compressed air, which will accelerate through the bypass created between the contracted sealing elements and the container inner wall. The contraction will be maintained and only cease with reduced speed and reduced piston extension, but fully cease with 0 piston extension, whereas the sealing capability between the piston sealing element and the container inner wall is recovered and fully re-established at the final position of the piston.

In an aspect, the invention provides a method of inserting a piston into a cylinder, e.g. a cylinder of an injector especially an injector for delivery pharmaceutical composition comprising a liquid drug. The method comprising the steps of:
providing an injector comprising a cylinder having an inner wall and an inner diameter and an outlet at an outlet end opposite an actuating end, and a stopper having a stopper body with an actuating surface opposite an outlet surface, an axial length between the actuating surface and the outlet surface, and a transverse diameter, which stopper body defines a cavity entrance having an access diameter, the stopper at an axial location from the actuating surface comprising a deformable sealing element surrounding the stopper body and having an outer diameter, which is larger than the transverse diameter, and the stopper comprising a piston cavity extending from the cavity entrance to a base of the piston cavity, the piston cavity defining a total cavity length, providing a piston extension rod having a diameter equal to or smaller than the access diameter and a length at least 5% larger than the total cavity length, the piston extension rod having an impact surface at an end of the piston extension rod, inserting the piston in the cylinder at the actuating end so that the deformable sealing element seals an annular gap between the stopper body and the inner wall of the cylinder, inserting the piston extension rod in the cavity entrance and impacting the impact surface with the base of the piston cavity at an accelerated movement of at least 400 mm/min, moving the piston extension rod in a longitudinal direction from the actuating end of the cylinder to the outlet end of the cylinder to deform the deformable sealing element and create a bypass for air between the deformable sealing element and the inner wall of the cylinder. The piston extension rod is inserted in the cavity entrance and impacting the impact surface with the base of the piston cavity at an accelerated movement of at least 400 mm/min. However, it is to be understood that it is accelerated movement of at least 400 mm/min that causes the deformation of the deformable sealing element and creates a bypass for air between the deformable sealing element and the inner wall of the cylinder. It is therefore contemplated and within the present invention that the piston extension rod is inserted into the cavity entrance at a low velocity, i.e. at a movement below 400 mm/min, for the impact surface to impact with the base of the piston cavity before increasing the velocity to reach an accelerated movement of at least 400 mm/min. Correspondingly, it is also possible to stop the movement of the piston extension rod after inserting into the cavity entrance. For example, the piston extension rod may be inserted into the cavity entrance at a high or low velocity, and the movement may then be stopped, e.g. regardless of how far into the cavity entrance the piston extension rod has been inserted. When the piston extension rod is inserted into the cavity entrance at a low velocity it is especially relevant that the piston extension rod does not have a part or section, e.g. a backstop, that can impact the surface of the piston having the entrance of the cavity. For example, the piston extension rod may have a backstop located at a distance from the impact surface of at least 10% longer than the piston cavity length, e.g. the distance from the impact surface to the backstop may be at least 20% longer than the piston cavity length or at least 30% longer than the piston cavity length. In an example, the piston extension rod does not have a backstop. In an example, the piston extension rod is inserted into the cavity entrance at a movement below 400 mm/min, before increasing the movement to an accelerated movement of at least 400 mm/min.

Thereby, the piston extension rod, i.e. at the impact surface, pushes the piston, i.e. via the impact surface, and moves the piston. The movement of the piston using the piston extension rod causes the deformation of the deformable sealing element thereby creating the bypass for air between the deformable sealing element and the inner wall of the cylinder. In the present context, the accelerated movement may also be referred to as a velocity, and the two terms may be used interchangeably. In general, an accelerated movement of at least 400 mm/min is sufficient to deform the deformable sealing element and create the bypass for air between the deformable sealing element and the inner wall of the cylinder. However, the larger the accelerated movement, the larger the deformation and also the bypass for air. In other examples, the accelerated movement may be in the range of 400 mm/min to 10,000 mm/min, e.g. 400 mm/min to 5,000 mm/min, 400 mm/min to 2,000 mm/min or 400 mm/min to 800 mm/min.

The access diameter is equal to or larger than the diameter of the piston extension rod. In the present context this means that the piston extension rod can be inserted into the piston via the cavity entrance. Thus, it is also contemplated that the access diameter is smaller than the diameter of the piston extension rod, e.g. the access diameter may be 5% smaller than the diameter of the piston extension rod. However, it is preferred that the access diameter is at least 5% larger than the diameter of the piston extension rod, although the exact ratio between the access diameter and the diameter of the piston extension rod is not important. When the piston extension rod has a diameter smaller than the access diameter, the difference in the diameters creates flexibility in the material of the piston so that the piston is more easily extended in the longitudinal direction of the cylinder, thus more easily creating the bypass for air between the deformable sealing element and the inner wall of the cylinder, than when there is no space between the piston extension rod and the material of the piston, e.g. when the access diameter is equal to or smaller than the diameter of the piston extension rod. The access diameter may for example be in the range of 10% larger than the diameter of the piston extension rod to 500% larger than the diameter of the piston extension rod, e.g. at least 10% larger than the diameter of the piston extension rod, or at least 20% larger, at least 30% larger, at least 50% larger, at least 100% larger, at least 200% larger, or at least 400% larger than the diameter of the piston extension rod.

The method is especially useful when the injector is an injector for delivery of a pharmaceutical composition comprising a liquid drug, so that the method allows a piston to be inserted in the injector and simultaneously remove air between the piston and the surface of the liquid drug. This is particularly relevant for prefilled syringes, in particular for prefilled syringes intended for delivery of small doses, e.g. of ophthalmic drugs.

The method in particular allows insertion of a piston into an injector i.e. the cylinder of an injector, without the need for lubrication, e.g. silicone lubrication. Silicone lubrication free injectors may have a piston made from a TPE, such as SEBS, SBS, etc. In a specific example, the stopper comprises a cavity at the axial location of the deformable sealing element, which cavity has a lateral extension larger than the access diameter of the cavity entrance. Such stoppers are described in WO 2019/185101. The presence of a cavity at the axial location of the deformable sealing element, e.g. a cavity having a lateral extension larger than the access diameter of the cavity entrance, allows an even greater flexibility thereby more easily creating the bypass for air. The piston may have a single deformable sealing element, and the impact between the piston extension rod and the impact surface at an accelerated movement of at least 400 mm/min can create the bypass for air. The piston may also have two or more deformable sealing elements. When the piston has two, or more, deformable sealing elements, each sealing element will abut the inner wall of the cylinder, and thereby the deformation of the piston caused by the impact of the piston extension rod with the base of the piston cavity will be larger than when the piston has a single deformable sealing element, so that the accelerated movement of at least 400 mm/min is also sufficient to create the bypass for air when the piston has two, or more, deformable sealing elements.

Besides the ability to bypass air from a PFS the invention introduces further derived significant advantages. The contraction of the sealing elements enables a low friction, and in some cases even 0 friction piston mounting without the usual strain and stress on sealing elements known from existing piston mounting systems. The invention renders vacuum redundant, including costs related to invest in and operate the vacuum system. The mounting speed can be significantly increased due to low friction mounting, hence enabling a larger annual output per time unit. The lower power consumption and higher output per time unit will have a significant positive impact on the CO₂ operating profile compared to both vent-tubing but also vacuum mounting. The appropriate piston extension rod speed may be chosen for each piston characteristic and PFS configuration and the piston extension rod speed range is at least 400mm/min and up to maximum speeds of the automated filling and mounting systems on which the piston extension rod is mounted.

The piston extension rod may also be functional for manual piston mounting, but is preferred as an integrated component for an automated piston mounting system e.g. a tabletop system or larger piston mounting system for multiple syringe filling and piston mounting lines.

For automated mounting the piston extension rod can be incorporated as a new component in a new or existing PFS mounting system, as a replacement for existing mounting components, e.g. rods and pins for vent-tubing, and assisted vacuum mounting.

Existing filling and mounting systems are relatively similar in order to comply with general standards including ISO11040, and where most common brands are Bausch & Ströbel, Bosch, Syntegon, Groninger, Optima Packaging, Kähle and Colanar.

The piston extension rod may be manufactured from any material as desired, e.g. a metal, preferably hardened steel, but can also be manufactured from plastic compounds considered suitable for the purpose.

The piston extension rod is compatible with elastomeric pistons comprising a cavity, however such pistons usually being within the Shore A durometer range for injectors. These pistons typically have a shore A value ranging from 40 - 75. The axial dimensions of the piston extension rod may vary relating to the specific piston with which it cooperates. While its length will be at least 5% more than the piston cavity measured from the internal cavity base to cavity opening along the container longitudinal axis, the piston extension rod width will vary with piston cavity diameter.

A reduced piston extension rod width at lowest piston extension rod surface impacting with piston cavity base will imply an increased extension, while an increased width will reduce the piston extension. The piston extension rod may incorporate a backstop limiting its stroke, which backstop can be implemented a number of ways including a plate interacting with the piston upper surface as a physical brake thereby restricting excessive extension of the piston.

The piston extension rod can be modified for each individual injector/piston configuration where piston size, material hardness, thickness, coating, number of sealing elements, total sealing area and container material are the factors, which may have an influence on the piston extension rod dimensions. The piston extension rod has the capability to achieve the appropriate piston extension by varying the speed, but also by extending its length to more than the 5% increase of the total piston cavity length.

The injector piston is an elastomeric component with at least one deformable sealing element but can be made of any suitable elastomeric material, such as natural rubbers, TPEs, styrene-butadiene block copolymers, polyisoprene, polybutadiene, ethylene propylene rubber, ethylene propylene diene rubber, silicone elastomers, fluoroelastomers, polyurethane elastomers, halobutyl rubbers and nitrile rubbers, and may furthermore be coated with a PTFE, or similar coating types for friction reduction.

The piston mounting is performed after the filling of the syringe, said syringe positioned vertically with container outlet in a downward position and filled with injectable liquid from its container opening in the upward position.

By example a standard glass 1ml long PFS has a nominal volume, which is smaller than the total container volume in order to accommodate the piston and a portion of the piston rod which is mounted in the piston after the piston mounting. The nominal volume represents a stroke of approximately 32mm and the residual stroke of approximately 21mm represents the amount of air on top of the liquid injectable, which must be bypassed for the piston to be mounted closest to the drug upper surface.

It is further contemplated that the method of the invention can also be used to mount a vial stopper in a vial. Thus, in another aspect the invention relates to a method of mounting a vial stopper in a vial. The vial stopper has a cavity generally as defined for the piston, and any feature relevant for the piston to be mounted in a cylinder of an injector is relevant for mounting a vial in a vial stopper.

Any embodiment of the invention may be used in any aspect of the invention, and any advantage for a specific embodiment applies equally when an embodiment is used in a specific aspect.

### Brief description of the drawings

Figure 1a shows a piston extension rod for use in the method of the invention;
Figure 1b shows a top view of a piston extension rod for use in the method of the invention;
Figure 2 shows an injector with a piston ready for mounting in the method of the invention;
Figure 3 shows an injector with a piston ready for mounting in the method of the invention;
Figure 4 shows an injector with a piston and a piston extension rod;
Figure 5 shows an injector with a piston being mounted with a piston extension rod in the method of the invention;
Figure 6 shows an injector with a piston mounted using a piston extension rod in the method of the invention;
Figure 7 shows a piston for mounting in the method of the invention;
Figure 8 shows an injector with a piston ready for mounting in the method of the invention;
Figure 9 shows an injector with a piston being mounting in the method of the invention;
Figure 10 shows an injector with a piston in its final position;
Figure 11 shows the steps of an example of the method of the invention;
Figure 12 depicts a modified vent tube equipment for use in the method of the invention;
Figure 13 shows parameters for an example of the method of the invention.

The invention is not limited to the embodiment/s illustrated in the drawings. Accordingly, it should be understood that where features mentioned in the appended claims are followed by reference signs, such signs are included solely for the purpose of enhancing the intelligibility of the claims and are in no way limiting on the scope of the claims.

The term "comprising" as used in this specification and claims means "consisting at least in part of". When interpreting statements in this specification and claims which include the term "comprising", other features besides the features prefaced by this term in each statement can also be present. Related terms such as "comprise" and "comprised" are to be interpreted in a similar manner.

### Detailed Description

The mounting process can be varied with each application. Automated mounting systems will often make a two-step mounting starting with a prepositioning for fixation of the piston just inside the most upper portion of the container often mounted by way of a separate tool followed by the actual downward movement and final positioning of the piston by a placement pin or rod. The piston extension rod is independent of the prepositioning of the piston, since the piston extension rod functions during the downward movement and final positioning of the piston and hence functions appropriately regardless of prepositioning configuration and any examples of prepositioning are without prejudice relating to the piston extension rod invention, but merely serve as explanatory.

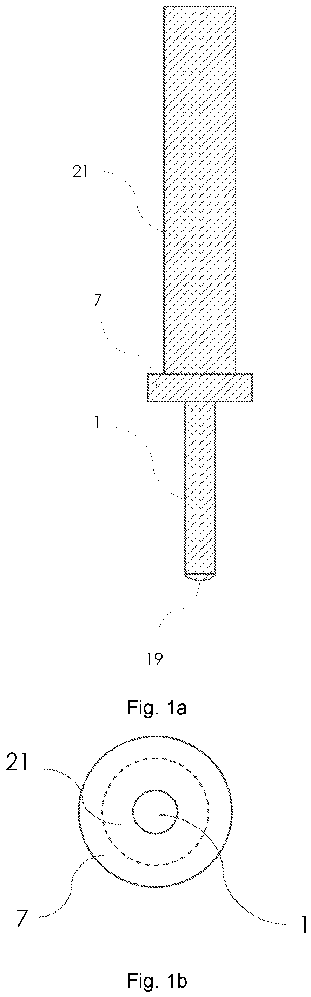

Fig. 1a and Fig. 1b show the piston extension rod 1 with a backstop 7 and connector 21 for mounting on automation machinery, and piston extension rod 1 rounded impact surface 19 for cooperation with piston cavity base 20 during piston positioning. The impact surface can have different geometry and instead of rounded can be flat or pointed rather than rounded, depending on piston cavity base 20 geometry. By reduced piston extension rod 1 width relative to the piston cavity base 20 the extension will be increased, while increased width relative to the piston cavity base 20 will result in decreased extension.

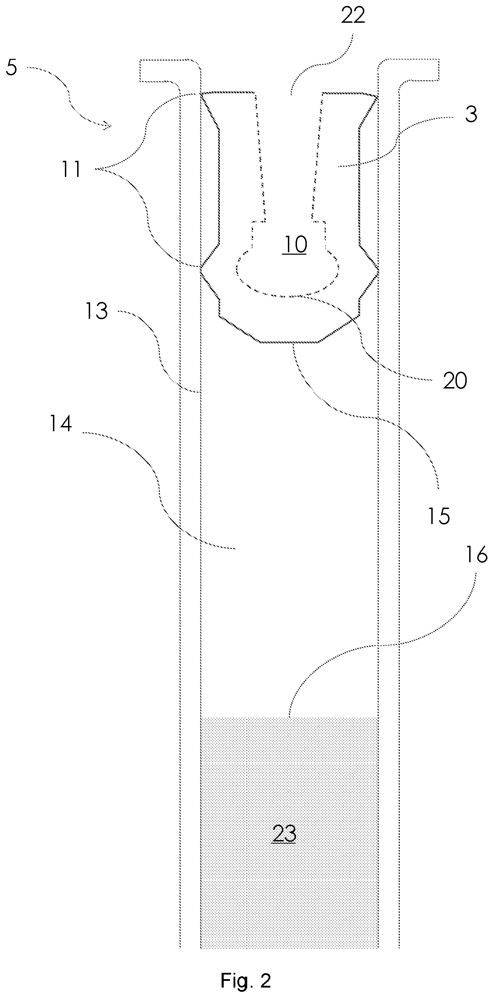

Fig. 2 shows the piston 3 after prepositioning inside the drug filled container upper portion 5 and air 14 between piston outlet surface 15 and liquid drug upper surface 16, and shows the cavity opening 22 and the piston cavity base 20 and the liquid drug 23. The piston outlet surface 15 may also be referred to as the piston front surface 15.

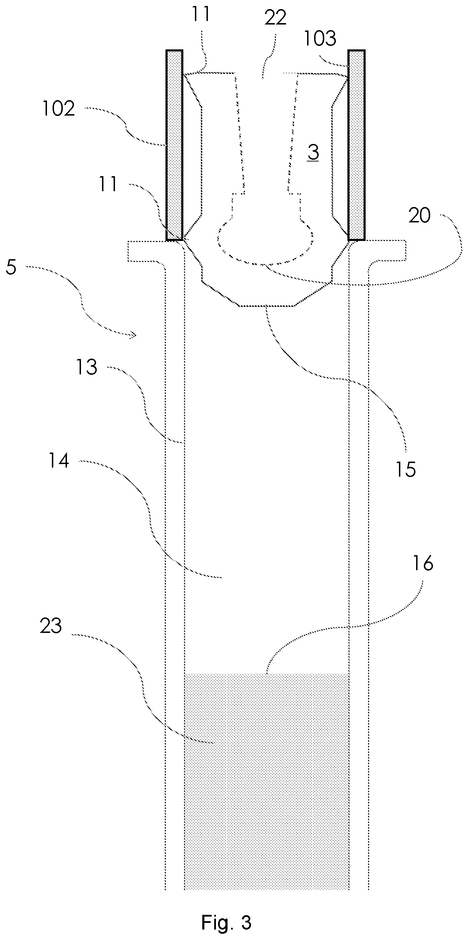

Fig. 3 shows an alternative piston 3 prepositioning outside and just above the container opening 22 and the piston 3 held in place by a dedicated tool, in Fig. 3 illustrated as an insertion tube 102. The insertion tube 102 has an inner wall 103.

Fig. 1 and Fig. 2 are hence merely examples of prepositioning. The prepositioning is prior to downward movement and final positioning by the piston extension rod 1 according to the invention.

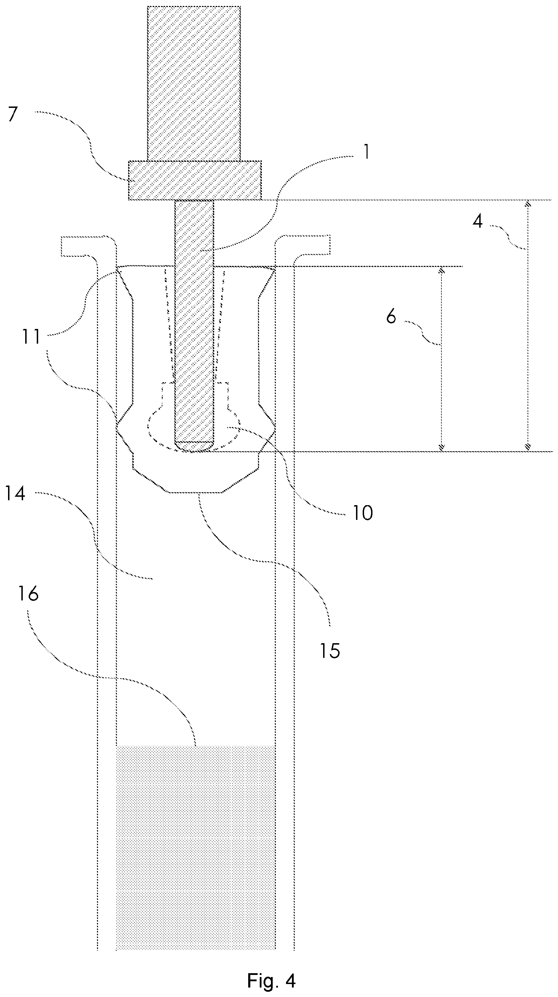

Fig. 4 shows the piston extension rod 1 configurated for final positioning of the piston 3 which piston is an SEBS piston with a cavity 10, as shown a broadened cavity 10, and a shore A hardness of 72 with two sealing elements 11. The piston extension rod 1 is seen positioned inside the prepositioned piston 3 during acceleration just at or before impact with piston cavity base 20.

The piston extension rod 1 has a length 4 exceeding the piston cavity length 6 by approximately 30% and in this configuration has a backstop 7 limiting the longitudinal extension of the piston 3.

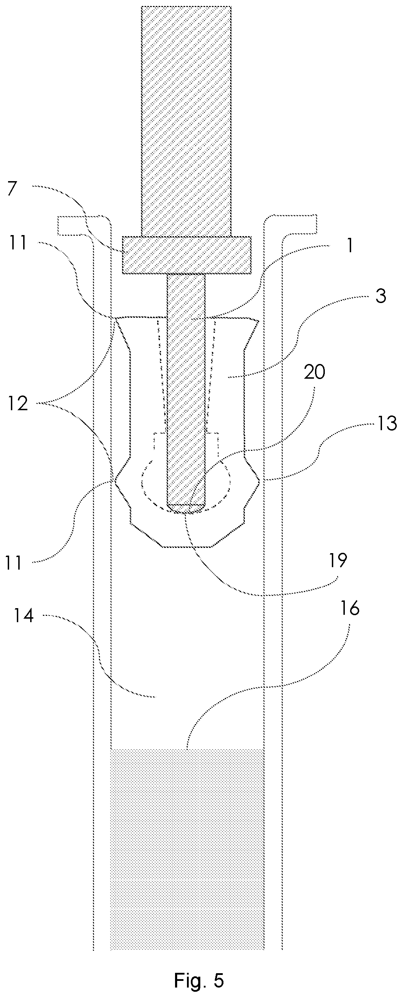

Fig. 5 shows the piston 3 in movement and extended after impact between piston extension rod 1 impact surface 19 and piston cavity base 20, which extended piston configuration is maintained during movement, e.g. continuous movement, of the piston extension rod 1 and piston. Shown is also the piston contraction as a function of the piston extension where piston sealing elements 11 are forced away from their sealing with the container inner wall 13 resulting in an open passage 12 between sealing elements 11 and container inner wall 13 for air to bypass.

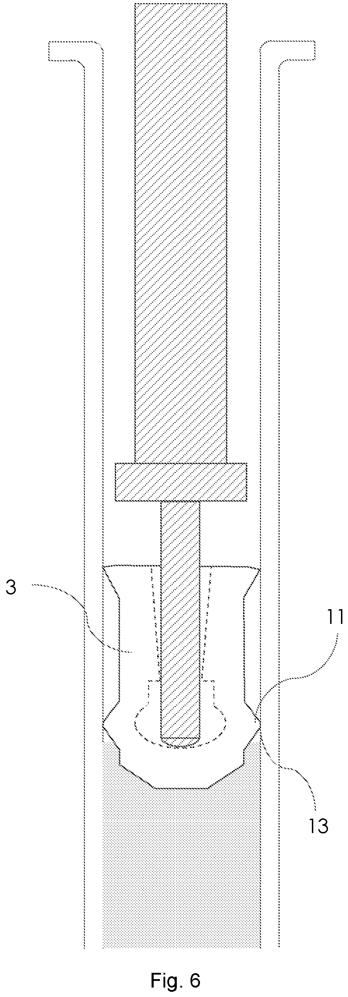

Fig. 6 shows the final positioning of the piston 3 where the piston extension 1 and contraction are ceased and the full sealing of piston sealing elements 11 are re-established towards the container inner wall 13.

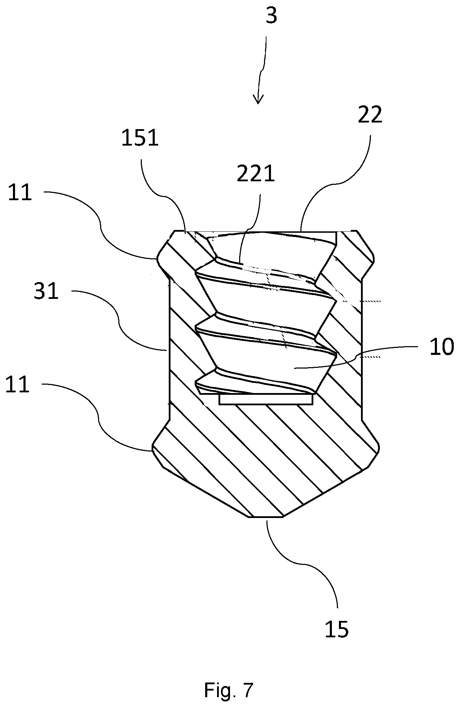

Fig. 7 shows an example of a piston 3 for mounting in the method of the invention. The piston 3 has a piston body 31 with and an actuating surface 151 and an outlet end 15, which actuating surface 151 comprises a cavity opening 22, and the cavity 10 has an internal helical thread 221.

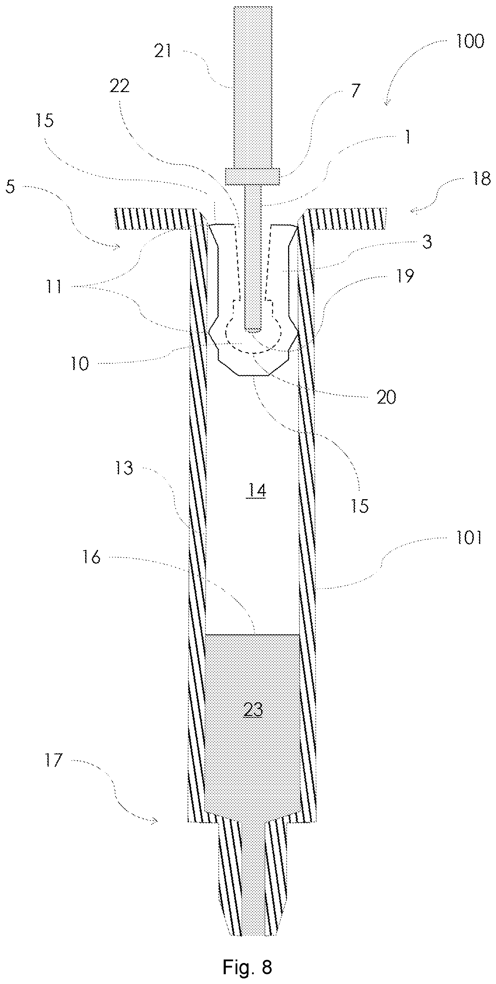

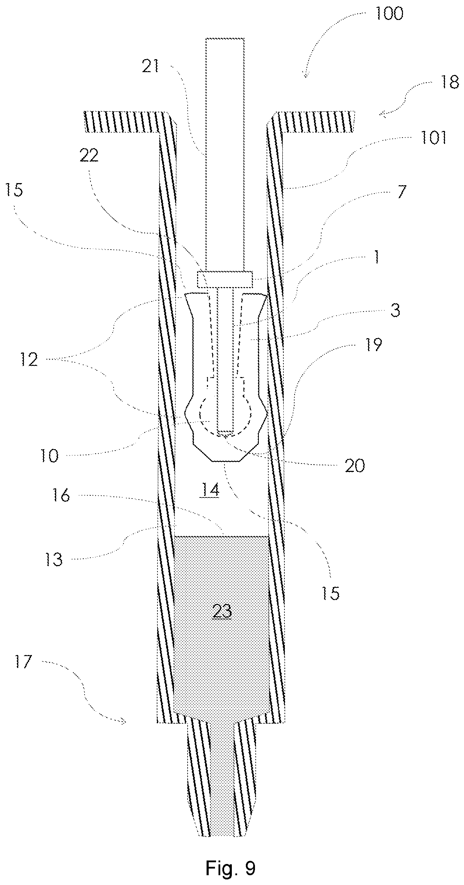

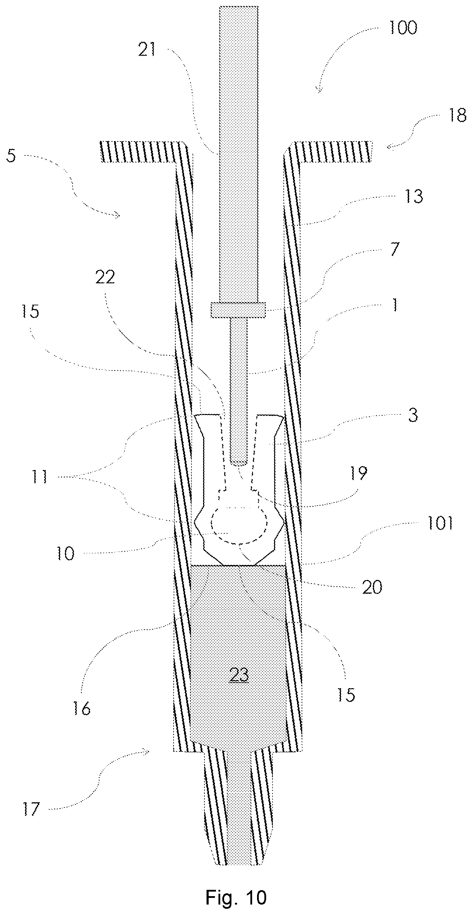

Fig. 8 to 10 show an injector 100 with a piston 3 illustrating the method of the invention. Figs. 8 to 10 show the cylinder 101 and illustrate the outlet end 17 of the cylinder and the actuating end of the cylinder 18. Thus, in Fig. 8, the piston 3 has been inserted in the cylinder 101 and is ready for mounting in the method. In Fig. 9, the piston cavity base 20 has been impacted with the impact surface 19 of the piston extension rod 1 to extend the piston 3 in the longitudinal dimension of the cylinder 101, whereby the piston 3 is contracted to create an open passage 12 between the deformable sealing elements 11 and the inner wall 13 of the cylinder 101 so that air 14 can leave the space between the piston 3 and the liquid drug upper surface 16. In Fig. 10, the piston 3 is at its final position so that the outlet surface 15 of the piston is in contact with the liquid drug in the cylinder 101.

### Example

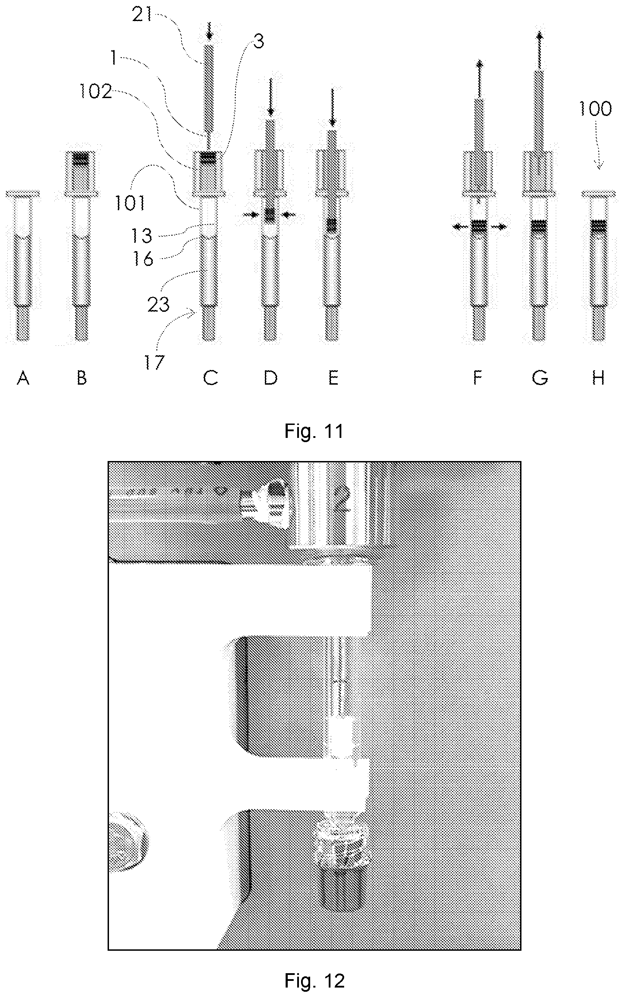

In an example, the method was performed in an appropriately modified, but otherwise conventional, vent tube equipment, where the vent tube was replaced with a simple fixture for holding the piston. The piston placed was placed relatively above the cylinder opening before the piston extension rod initiates the deformation. Specifically, the piston was placed in an insertion tube, in this case of a slightly larger inner diameter than the inner diameter of the syringe. The steps of the process are shown in Fig. 11 where the reference numerals of item C are understood to be relevant for items A, B and D to H. A shows the prefilled cylinder 101 prior to insertion of the piston 3, B shows the piston 3 inserted in the insertion tube 102. C shows the piston extension rod 1 extending from a connector 21 and being inserted in the cavity (not shown in Fig. 11) of the piston 3. D shows the piston extension rod 1 being moved at the intended velocity to illustrate the contraction of the deformable sealing elements (not shown in Fig. 11) with the arrows indicating the direction of movement of the piston extension rod 1 and the contraction, respectively. E shows the piston 3 having been moved to its final position prior to stopping the movement of the piston extension rod 1. F and G show the piston 3 at its final position with the deformable sealing elements abutting the inner wall 13 of the cylinder and the piston extension rod 1 being removed. H shows the injector 100 in its final, prefilled stage. The set-up is depicted in Fig. 12. In the set-up, a mounting velocity of 300 mm/s was used, and acceleration and deacceleration was performed at a minimum of 10 mm/s².

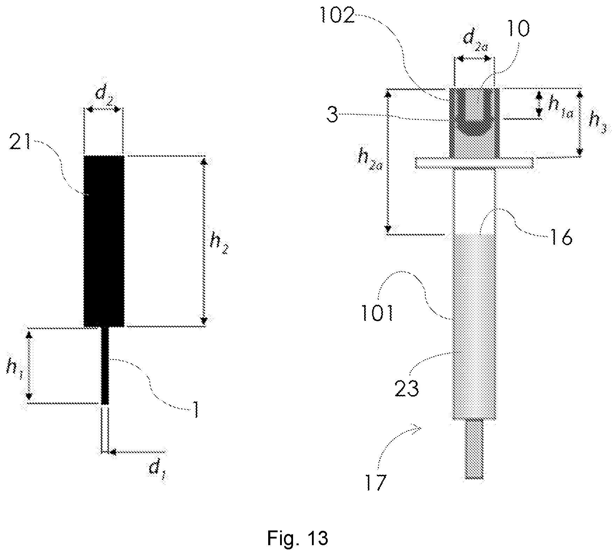

Relevant parameters are provided in Table 1 with reference to Fig. 13.

**Table 1**

| Plunger/ syringe size | *d₁* | *d₂* | *h₁* | *h₂* | *h₃* |
|---|---|---|---|---|---|
| 0.5 ml | Ø1.2 mm | | *h₁* > *h₁ₐ* | | |
| 1.0 mL long | Ø1.5 mm | *d₂* < *d₂ₐ* | ≥*10* mm *(recommen ded)* | *h₂* > *h₂ₐ - h₁* | *>15 mm* |
| 2.25 mL (1 to 3 mL) | Ø2.5 mm | | | | |

The method of the invention was thus applied via high-speed industrial machinery with speeds up toward 60,000 units/hour, although the method can also be performed using smaller desktop equipment in isolators for small batch or customised applications.

### Reference signs list

- 1: Piston extension rod
- 100: Injector
- 101: Cylinder
- 102: Insertion tube
- 103: Inner wall of the insertion tube
- 10: Cavity
- 11: Piston deformable sealing element
- 12: Open passage
- 13: Inner wall of the cylinder
- 14: Air
- 15: Piston outlet surface
- 151: Actuating surface of the piston
- 16: Liquid drug upper surface
- 17: Outlet end of the cylinder
- 18: Actuating end of the cylinder
- 19: Impact surface
- 20: Piston cavity base
- 21: Connector
- 22: Cavity opening
- 221: Internal helical thread
- 23: Liquid drug
- 3: Piston
- 31: piston body
- 4: Length
- 5: Container upper portion
- 6: Piston cavity length
- 7: Backstop

## Claims

1. A method for mounting a piston (3) in a prefilled injector (100), the method comprising the steps of:
providing an injector (100) comprises a cylinder (101) having a longitudinal axis and an inner wall (103), and a liquid drug defining a drug upper surface (16),
providing a piston (3) having a deformable sealing element (11) and a cavity (10) extending from a piston cavity opening (22) to a piston cavity base (20) to define a total piston cavity length (6),
inserting the piston (3) in the cylinder (101) so that the deformable sealing element (11) abuts the inner wall (103) of the cylinder (101) and seals an annular gap between the piston (3) and the inner wall (103) of the cylinder (101),
providing a piston extension rod (1) having a total length (4) of at least 5% more than the total cavity length (6), and an impact surface (19),
**characterised in the steps of:**
inserting the piston extension rod (1) in the cavity (10) of the piston (3) and impacting the piston cavity base (20) with the impact surface (19) at a velocity of at least 400 mm/min to extend the piston (3) along the cylinder longitudinal axis and cause a contraction of the deformable sealing element (11) to create a bypass for air between the deformable sealing element (11) and the inner wall (103) of the cylinder, and
maintaining the velocity of the piston extension rod (1) to move the piston (3) to a final piston position.

2. The method for mounting a piston (3) in a prefilled injector (100) according to claim 1, wherein the piston (3) comprises a deformable sealing element (11) at an axial location from the piston cavity opening (22), and a broadened cavity section at the axial location of the deformable sealing element (11), which broadened cavity section has an axial extension in the range of 5% and 70% of the total piston cavity length (6), and which broadened cavity section has a lateral extension that is larger than the piston cavity opening (22), and wherein the lateral extension of the broadened cavity section is in the range of 50% to 90% of the outer diameter of the deformable sealing element (11).

3. The method for mounting a piston (3) in a prefilled injector (100) according to claim 2, wherein the piston (3) has a tubular section for receiving the piston extension rod (1), which tubular section extends from the piston cavity opening (22) to the broadened cavity section.

4. The method for mounting a piston (3) in a prefilled injector (100) according to any one of claims 1 to 3, wherein the piston (3) has an actuating surface opposite (151) an outlet surface (15), and wherein the outlet surface (15) of the piston (3) is in contact with the liquid drug (23) in the cylinder (101) at the final piston position.

5. The method for mounting a piston (3) in a prefilled injector (100) according to any one of claims 1 to 4, wherein the piston extension rod (1) has a length (4) that is 5% to 50% longer than the total piston cavity length (6).

6. The method for mounting a piston (3) in a prefilled injector (100) according to any one of claims 1 to 5, wherein the piston extension rod (1) has an outward protrusion and/or an inward groove in its longitudinal axis suitable for interaction with the piston cavity (10).

7. The method for mounting a piston (3) in a prefilled injector (100) according to any one of claims 1 to 6, wherein at least one of the deformable sealing element (11), and the piston (3) and the deformable sealing element (11) is made from a thermoplastic elastomer and has a Shore A hardness in the range of 40 to 75.

8. A kit of parts comprising an injector (100) comprising a cylinder (101) having a longitudinal axis and an inner wall (103);
a piston (3) having a piston body (31) with an actuating surface (151) opposite an outlet surface (15) and a transverse diameter, which piston (3), at an axial location from the actuating surface (151), comprises a deformable sealing element (11) surrounding the piston body and having an outer diameter, which is larger than the transverse diameter, the piston (3) having a cavity (10) extending from a piston cavity opening (22) to a piston cavity base (20) to define a total piston cavity length (6), the cavity (10) comprising a tubular section extending from the piston cavity opening (22) and having an access diameter, and a broadened cavity section at the axial location of the deformable sealing element (11) and having an axial extension in the range of 5% and 50% of the total piston cavity length (6), and which broadened cavity section has a lateral extension that is larger than the access diameter and in the range of 50% to 90% of the outer diameter of the deformable sealing element (11); and
a piston extension rod (1) having a diameter equal to or smaller than the access diameter and a length (4) at least 5% larger than the total cavity length (6), and an impact surface (19).

9. The kit of parts according to claim 8, wherein the piston extension rod (1) comprises a backstop (7) located at an end opposite the impact surface (19), which backstop (7) has a lateral size larger than the piston cavity opening (22) and smaller than the inner diameter of the cylinder (101).

10. The kit of parts according to any one of claims 8 or 9, wherein the tubular section extends from the piston cavity opening (22) to the broadened cavity section.

11. The kit of parts according to any one of claims 8 to 10, wherein the piston extension rod (1) has a diameter, and the access diameter is in the range of 10% to 500% larger than the diameter of the piston extension rod (1).

12. The kit of parts according to any one of claims 8 to 11, wherein the piston extension rod (1) has a length (4) that is 5% to 50% longer than the total piston cavity length (6).

13. The kit of parts according to any one of claims 8 to 12, wherein the piston extension rod (1) has an outward protrusion and/or an inward groove in its longitudinal axis suitable for interaction with the piston cavity (10).

14. The kit of parts according to any one of claims 8 to 13, wherein at least one of the deformable sealing element (11), and the piston (3) and the deformable sealing element (11) is made from a thermoplastic elastomer, and have a Shore A hardness in the range of 40 to 75.

15. The kit of parts according to any one of claims 8 to 14, wherein the piston extension rod (1) extends from a connector (21).

## Patentansprüche

1. Verfahren zum Montieren eines Kolbens (3) in einem vorgefüllten Injektor (100), wobei das Verfahren die Schritte umfasst:
Bereitstellen eines Injektors (100) umfassend einen Zylinder (101) mit einer Längsachse und einer Innenwand (103) und ein flüssiges Arzneimittel, das eine obere Arzneimitteloberfläche (16) definiert,
Bereitstellen eines Kolbens (3) mit einem verformbaren Dichtungselement (11) und einem Hohlraum (10), der von einer Kolbenhohlraumöffnung (22) zu einem Kolbenhohlraumboden (20) verläuft, um eine Gesamt-Kolbenhohlraumlänge (6) zu definieren,
Einführen des Kolbens (3) in den Zylinder (101), so dass das verformbare Dichtungselement (11) an der Innenwand (103) des Zylinders (101) anliegt und einen Ringspalt zwischen dem Kolben (3) und der Innenwand (103) des Zylinders (101) abdichtet,
Bereitstellen eines Kolbenverlängerungsstabs (1) mit einer Gesamtlänge (4) von wenigstens 5 % mehr als die Gesamt-Hohlraumlänge (6) und einer Stoßfläche (19),
**gekennzeichnet durch** die Schritte:
Einführen des Kolbenverlängerungsstabs (1) in den Hohlraum (10) des Kolbens (3) und Stoßen auf den Kolbenhohlraumboden (20) mit der Stoßfläche (19) mit einer Geschwindigkeit von wenigstens 400 mm/min, um den Kolben (3) entlang der Zylinderlängsachse zu verlängern und eine Kontraktion des verformbaren Dichtungselements (11) zu bewirken, um einen Umgehungsweg für Luft zwischen dem verformbaren Dichtungselement (11) und der Innenwand (103) des Zylinders zu schaffen, und
Beibehalten der Geschwindigkeit des Kolbenverlängerungsstabs (1), um den Kolben (3) in eine Kolben-Endposition zu bewegen.

2. Verfahren zum Montieren eines Kolbens (3) in einem vorgefüllten Injektor (100) nach Anspruch 1, wobei der Kolben (3) ein verformbares Dichtungselement (11) an einer axialen Stelle von der Kolbenhohlraumöffnung (22) und einen verbreiterten Hohlraumabschnitt an der axialen Stelle des verformbaren Dichtungselements (11) umfasst, wobei der verbreiterte Hohlraumabschnitt eine axiale Ausdehnung in dem Bereich von 5 % bis 70 % der Gesamt-Kolbenhohlraumlänge (6) aufweist und der verbreiterte Hohlraumabschnitt eine seitliche Ausdehnung aufweist, die größer als die Kolbenhohlraumöffnung (22) ist, und wobei die seitliche Ausdehnung des verbreiterten Hohlraumabschnitts in dem Bereich von 50 % bis 90 % des Außendurchmessers des verformbaren Dichtungselements (11) liegt.

3. Verfahren zum Montieren eines Kolbens (3) in einem vorgefüllten Injektor (100) nach Anspruch 2, wobei der Kolben (3) einen rohrförmigen Abschnitt zur Aufnahme des Kolbenverlängerungsstabs (1) aufweist, wobei der rohrförmige Abschnitt von der Kolbenhohlraumöffnung (22) zu dem verbreiterten Hohlraumabschnitt verläuft.

4. Verfahren zum Montieren eines Kolbens (3) in einem vorgefüllten Injektor (100) nach einem der Ansprüche 1 bis 3, wobei der Kolben (3) eine Betätigungsfläche gegenüber (151) einer Austrittsfläche (15) aufweist und wobei sich die Austrittsfläche (15) des Kolbens (3) an der Kolben-Endposition in Kontakt mit dem flüssigen Arzneimittel (23) in dem Zylinder (101) befindet.

5. Verfahren zum Montieren eines Kolbens (3) in einem vorgefüllten Injektor (100) nach einem der Ansprüche 1 bis 4, wobei der Kolbenverlängerungsstab (1) eine Länge (4) aufweist, die 5 % bis 50 % länger als die Gesamt-Kolbenhohlraumlänge (6) ist.

6. Verfahren zum Montieren eines Kolbens (3) in einem vorgefüllten Injektor (100) nach einem der Ansprüche 1 bis 5, wobei der Kolbenverlängerungsstab (1) einen nach außen gerichteten Vorsprung und/oder eine nach innen gerichtete Nut in seiner Längsachse aufweist, der/die zum Zusammenwirken mit dem Kolbenhohlraum (10) geeignet ist.

7. Verfahren zum Montieren eines Kolbens (3) in einem vorgefüllten Injektor (100) nach einem der Ansprüche 1 bis 6, wobei wenigstens eines von dem verformbaren Dichtungselement (11) und dem Kolben (3) und dem verformbaren Dichtungselement (11) aus einem thermoplastischen Elastomer besteht und eine Shore-A-Härte in dem Bereich von 40 bis 75 aufweist.

8. Teilesatz umfassend einen Injektor (100), der einen Zylinder (101) mit einer Längsachse und einer Innenwand (103) umfasst;
einen Kolben (3) mit einem Kolbenkörper (31) mit einer einer Betätigungsfläche (151) gegenüber einer Austrittsfläche (15) und einem Querdurchmesser, wobei der Kolben (3) an einer axialen Stelle von der Betätigungsfläche (151) ein den Kolbenkörper umgebendes verformbares Dichtungselement (11) mit einem Außendurchmesser umfasst, der größer als der Querdurchmesser ist, wobei der Kolben (3) einen Hohlraum (10) aufweist, der von einer Kolbenhohlraumöffnung (22) zu einem Kolbenhohlraumboden (20) verläuft, um eine Gesamt-Kolbenhohlraumlänge (6) zu definieren, wobei der Hohlraum (10) einen rohrförmigen Abschnitt aufweist, der von der Kolbenhohlraumöffnung (22) ausgeht und einen Zugangsdurchmesser aufweist, und einen verbreiterten Hohlraumabschnitt an der axialen Stelle des verformbaren Dichtungselements (11), der eine axiale Ausdehnung in dem Bereich von 5 % und 50 % der Gesamt-Kolbenhohlraumlänge (6) aufweist, und der verbreiterte Hohlraumabschnitt eine seitliche Ausdehnung aufweist, die größer als der Zugangsdurchmesser ist und in dem Bereich von 50 % bis 90 % des Außendurchmessers des verformbaren Dichtungselements (11) liegt; und
einen Kolbenverlängerungsstab (1) mit einem Durchmesser von gleich oder kleiner als der Zugangsdurchmesser und einer Länge (4), die wenigstens 5 % größer als die Gesamt-Hohlraumlänge (6) ist, und einer Stoßfläche (19).

9. Teilesatz nach Anspruch 8, wobei der Kolbenverlängerungsstab (1) einen Rückanschlag (7) umfasst, der an einem der Stoßfläche (19) gegenüberliegenden Ende angeordnet ist, wobei der Rückanschlag (7) eine seitliche Größe aufweist, die größer als die Kolbenhohlraumöffnung (22) und kleiner als der Innendurchmesser des Zylinders (101) ist.

10. Teilesatz nach einem der Ansprüche 8 oder 9, wobei der rohrförmige Abschnitt von der Kolbenhohlraumöffnung (22) zu dem erweiterten Hohlraumabschnitt verläuft.

11. Teilesatz nach einem der Ansprüche 8 bis 10, wobei der Kolbenverlängerungsstab (1) einen Durchmesser aufweist und der Zugangsdurchmesser in dem Bereich von 10 % bis 500 % größer als der Durchmesser des Kolbenverlängerungsstabs (1) ist.

12. Teilesatz nach einem der Ansprüche 8 bis 11, wobei der Kolbenverlängerungsstab (1) eine Länge (4) aufweist, die 5 % bis 50 % länger als die Gesamt-Kolbenhohlraumlänge (6) ist.

13. Teilesatz nach einem der Ansprüche 8 bis 12, wobei der Kolbenverlängerungsstab (1) einen nach außen gerichteten Vorsprung und/oder eine nach innen gerichtete Nut in seiner Längsachse aufweist, der/die zur Wechselwirkung mit dem Kolbenhohlraum (10) geeignet ist.

14. Teilesatz nach einem der Ansprüche 8 bis 13, wobei wenigstens eines von dem verformbaren Dichtungselement (11) und dem Kolben (3) und dem verformbaren Dichtungselement (11) aus einem thermoplastischen Elastomer besteht und eine Shore-A-Härte in dem Bereich von 40 bis 75 aufweist.

15. Teilesatz nach einem der Ansprüche 8 bis 14, wobei der Kolbenverlängerungsstab (1) von einem Verbinder (21) ausgeht.

## Revendications

1. Procédé de montage d'un piston (3) dans un injecteur prérempli (100), le procédé comprenant les étapes de :
la fourniture d'un injecteur (100) comprenant un cylindre (101) ayant un axe longitudinal et une paroi interne (103), et un médicament liquide définissant une surface supérieure de médicament (16),
la fourniture d'un piston (3) ayant un élément d'étanchéité déformable (11) et une cavité (10) s'étendant depuis une ouverture de cavité de piston (22) jusqu'à une base de cavité de piston (20) pour définir une longueur totale de cavité de piston (6),
l'insertion du piston (3) dans le cylindre (101) de sorte que l'élément d'étanchéité déformable (11) soit en butée contre la paroi interne (103) du cylindre (101) et obture un espace annulaire entre le piston (3) et la paroi interne (103) du cylindre (101),
la fourniture d'une tige d'extension de piston (1) ayant une longueur totale (4) supérieure d'au moins 5 % à la longueur totale de cavité (6), et une surface d'impact (19),
**caractérisé par** les étapes de :
l'insertion de la tige d'extension de piston (1) dans la cavité (10) du piston (3) et l'impact de la base de cavité de piston (20) avec la surface d'impact (19) à une vitesse d'au moins 400 mm/min pour allonger le piston (3) le long de l'axe longitudinal du cylindre et provoquer une contraction de l'élément d'étanchéité déformable (11) afin de créer une dérivation pour l'air entre l'élément d'étanchéité déformable (11) et la paroi interne (103) du cylindre, et
le maintien de la vitesse de la tige d'extension de piston (1) pour déplacer le piston (3) jusqu'à une position finale de piston.

2. Procédé de montage d'un piston (3) dans un injecteur prérempli (100) selon la revendication 1, le piston (3) comprenant un élément d'étanchéité déformable (11) à un emplacement axial par rapport à l'ouverture de cavité de piston (22), et une section de cavité élargie à l'emplacement axial de l'élément d'étanchéité déformable (11), laquelle section de cavité élargie ayant une étendue axiale comprise entre 5 % et 70 % de la longueur totale de cavité de piston (6), et laquelle section de cavité élargie ayant une étendue latérale supérieure à l'ouverture de cavité de piston (22), et l'étendue latérale de la section de cavité élargie étant comprise entre 50 % et 90 % du diamètre extérieur de l'élément d'étanchéité déformable (11).

3. Procédé de montage d'un piston (3) dans un injecteur prérempli (100) selon la revendication 2, le piston (3) ayant une section tubulaire pour recevoir la tige d'extension de piston (1), laquelle section tubulaire s'étendant depuis l'ouverture de cavité de piston (22) jusqu'à la section de cavité élargie.

4. Procédé de montage d'un piston (3) dans un injecteur prérempli (100) selon l'une quelconque des revendications 1 à 3, le piston (3) ayant une surface d'actionnement (151) opposée à une surface de sortie (15), et la surface de sortie (15) du piston (3) étant en contact avec le médicament liquide (23) dans le cylindre (101) à la position finale de piston.

5. Procédé de montage d'un piston (3) dans un injecteur prérempli (100) selon l'une quelconque des revendications 1 à 4, la tige d'extension de piston (1) ayant une longueur (4) supérieure de 5 % à 50 % à la longueur totale de cavité de piston (6).

6. Procédé de montage d'un piston (3) dans un injecteur prérempli (100) selon l'une quelconque des revendications 1 à 5, la tige d'extension de piston (1) ayant une saillie vers l'extérieur et/ou une rainure vers l'intérieur dans son axe longitudinal, appropriées pour coopérer avec la cavité de piston (10).

7. Procédé de montage d'un piston (3) dans un injecteur prérempli (100) selon l'une quelconque des revendications 1 à 6, au moins l'un parmi l'élément d'étanchéité déformable (11), et le piston (3) et l'élément d'étanchéité déformable (11) étant réalisé à partir d'un élastomère thermoplastique et ayant une dureté Shore A comprise entre 40 et 75.

8. Kit de pièces comprenant un injecteur (100) comprenant un cylindre (101) ayant un axe longitudinal et une paroi interne (103) ;
un piston (3) ayant un corps de piston (31) avec une surface d'actionnement (151) opposée à une surface de sortie (15) et un diamètre transversal, lequel piston (3), à un emplacement axial par rapport à la surface d'actionnement (151), comprend un élément d'étanchéité déformable (11) entourant le corps de piston et ayant un diamètre extérieur, qui est supérieur au diamètre transversal, le piston (3) ayant une cavité (10) s'étendant depuis une ouverture de cavité de piston (22) jusqu'à une base de cavité de piston (20) pour définir une longueur totale de cavité de piston (6), la cavité (10) comprenant une section tubulaire s'étendant depuis l'ouverture de cavité de piston (22) et ayant un diamètre d'accès, et une section de cavité élargie à l'emplacement axial de l'élément d'étanchéité déformable (11) et ayant une étendue axiale comprise entre 5 % et 50 % de la longueur totale de cavité de piston (6), et laquelle section de cavité élargie a une étendue latérale supérieure au diamètre d'accès et comprise entre 50 % et 90 % du diamètre extérieur de l'élément d'étanchéité déformable (11) ; et
une tige d'extension de piston (1) ayant un diamètre égal ou inférieur au diamètre d'accès et une longueur (4) supérieure d'au moins 5 % à la longueur totale de cavité (6), et une surface d'impact (19).

9. Kit de pièces selon la revendication 8, la tige d'extension de piston (1) comprenant une butée arrière (7) située à une extrémité opposée à la surface d'impact (19), laquelle butée arrière (7) ayant une taille latérale supérieure à l'ouverture de cavité de piston (22) et inférieure au diamètre intérieur du cylindre (101).

10. Kit de pièces selon l'une quelconque des revendications 8 ou 9, la section tubulaire s'étendant depuis l'ouverture de cavité de piston (22) jusqu'à la section de cavité élargie.

11. Kit de pièces selon l'une quelconque des revendications 8 à 10, la tige d'extension de piston (1) ayant un diamètre, et le diamètre d'accès étant supérieur de 10 % à 500 % au diamètre de la tige d'extension de piston (1).

12. Kit de pièces selon l'une quelconque des revendications 8 à 11, la tige d'extension de piston (1) ayant une longueur (4) supérieure de 5 % à 50 % à la longueur totale de cavité de piston (6).

13. Kit de pièces selon l'une quelconque des revendications 8 à 12, la tige d'extension de piston (1) ayant, dans son axe longitudinal, une saillie vers l'extérieur et/ou une rainure vers l'intérieur appropriées pour coopérer avec la cavité de piston (10).

14. Kit de pièces selon l'une quelconque des revendications 8 à 13, au moins l'un parmi l'élément d'étanchéité déformable (11), et le piston (3) et l'élément d'étanchéité déformable (11) étant réalisé à partir d'un élastomère thermoplastique, et ayant une dureté Shore A comprise entre 40 et 75.

15. Kit de pièces selon l'une quelconque des revendications 8 à 14, la tige d'extension de piston (1) s'étendant depuis un connecteur (21).
